(19) Europäisches Patentamt
European Patent Office
Office européen des brevets

(11) EP 1 946 744 A1

(12) # DEMANDE DE BREVET EUROPEEN

(43) Date de publication:
**23.07.2008 Bulletin 2008/30**

(21) Numéro de dépôt: **07123588.1**

(22) Date de dépôt: **19.12.2007**

(51) Int Cl.:
***A61K 8/89*** *(2006.01)* ***A61Q 5/00*** *(2006.01)*
***A61Q 5/12*** *(2006.01)* ***A61Q 5/10*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **20.12.2006 FR 0655726**

(71) Demandeur: **L'Oréal**
**75008 Paris (FR)**

(72) Inventeurs:
- **Brun, Gaëlle**
**75015 Paris (FR)**
- **Gourlaouen, Luc**
**92600 Asnieres (FR)**

(74) Mandataire: **Wattremez, Catherine et al**
**L'OREAL**
**River Plaza - DIPI**
**25-29 Quai Aulagnier**
**92665 Asnières-sur-Seine (FR)**

(54) **Traitement de fibres capillaires à partir d'une composition comprenant des pigments et des composés siliconés réactifs**

(57) La présente invention a pour objet un procédé de traitement de fibres capillaires consistant à appliquer une composition comprenant au moins un pigment, au moins un composé X, au moins un composé Y, au moins l'un de ces composés étant siliconé, lesdits composés X et Y étant par ailleurs susceptibles de réagir ensemble par une réaction d'hydrosilylation, de condensation ou de réticulation.

EP 1 946 744 A1

Printed by Jouve, 75001 PARIS (FR)

## Description

La présente invention a pour objet un procédé de traitement de fibres capillaires consistant à appliquer une composition comprenant au moins un pigment, au moins un composé X, au moins un composé Y, au moins l'un de ces composés étant siliconé, lesdits composés X et Y étant par ailleurs susceptibles de réagir ensemble par une réaction d'hydrosilylation, de condensation ou de réticulation.

Dans le domaine de la coloration des fibres capillaires, il est connu de mettre en oeuvre différentes techniques, plus particulièrement à partir de colorants directs ou de pigments pour des colorations non permanentes ou de précurseurs de colorant pour des colorations permanentes.

La coloration non permanente ou coloration directe consiste à teindre les fibres avec des compositions tinctoriales contenant des colorants directs. Ces colorants sont des molécules colorées et colorantes solubles ayant une affinité pour les cheveux. Ils sont appliqués sur les fibres pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Il est aussi connu de teindre les cheveux de façon permanente par la coloration d'oxydation. Cette technique de coloration consiste à appliquer sur les fibres, une composition contenant des précurseurs de colorants tels que des bases d'oxydation et des coupleurs. Ces précurseurs sous l'action d'un agent oxydant vont former l'espèce ou les espèces colorées par une réaction de condensation oxydative, à l'intérieur de la fibre même.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

De plus pour être visible sur cheveux foncés, ces deux techniques de coloration nécessitent souvent un éclaircissement préalable ou simultané des fibres. Cette étape d'éclaircissement est mise en oeuvre avec un agent oxydant tel que le peroxyde d'hydrogène ou de persels. L'inconvénient réside dans le fait que cette étape entraîne à la longue une dégradation des cheveux, ce qui altère leurs propriétés cosmétiques. Ils ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. On procède en général en appliquant sur les fibres, une composition comprenant une dispersion de particules d'un polymère filmogène comprenant un ou plusieurs pigments aussi dispersés. Les colorations obtenues par ce mode de coloration présentent cependant l'inconvénient d'avoir une faible résistance aux shampooings.

L'un des buts de la présente invention est de fournir un procédé de coloration capillaire, qui permettent d'obtenir des colorations visibles sur cheveux foncés sans qu'il soit nécessaire d'éclaircir ou de décolorer les fibres et qui présentent une bonne résistance aux shampooings.

Ainsi, la présente invention a pour objet un procédé de traitement de fibres capillaires, dans lequel on applique sur les fibres une composition comprenant au moins un pigment, au moins un composé X, au moins un composé Y, au moins un des composés X ou Y étant siliconé, lesdits composés X et Y étant susceptibles de réagir ensemble par une réaction d'hydrosilylation, ou par une réaction de condensation ou par une réaction de réticulation en présence d'un peroxyde, lorsqu'ils sont mis en contact les uns avec les autres.

Le procédé selon l'invention permet notamment d'obtenir un gainage des fibres qui est rémanent à plusieurs shampooings.

De plus, on a constaté de façon surprenante que les cheveux pouvaient être coiffés sans problème.

Un autre avantage du traitement selon l'invention est que l'on peut obtenir des effets optiques particuliers tels des effets métalliques, interférentiels, ce qui n'est en général pas possible avec les procédés de coloration directe ou d'oxydation.

Dans ce qui va suivre, les bornes encadrant un domaine de valeurs sont comprises dans ce domaine à moins qu'une indication contraire ne soit prévue.

Comme cela a été indiqué auparavant, la composition appliquée sur les fibres comprend au moins un composé X, au moins un composé Y, dont l'un au moins est un composé siliconé, et au moins un pigment.

### Composés X et Y

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO- , -COO- , -OH , $-NH_2$, -NH-,-NR-, $-SO_3H$, $-SO_3^-$, $-OCH_2CH_2-$, $-O-CH_2CH_2CH_2-$, $-O-CH_2CH(CH_3)-$, $-NR_3^+$,

$-SH$, $-NO_2$, I, Cl, Br, -CN, $-PO_4{}^{3-}$, -CONH- , -CONR-, $-CONH_2$, -CSNH-, $-SO_2-$ , -SO-, $-SO_2NH-$, -NHCO- , $-NHSO_2-$ , -NHCOO-, -OCONH-, -NHCSO- et -OCSNH-, R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique avantageusement en présence d'un catalyseur, par une réaction d'hydrosilylation ou une réaction de condensation, ou une réaction de réticulation en présence d'un peroxyde.

## 1- Composés X et Y susceptibles de réagir par hydrosilylation

Selon un mode de réalisation, les composés X et Y sont susceptibles de réagir par hydrosilylation, cette réaction pouvant être de manière simplifiée schématisée comme suit :

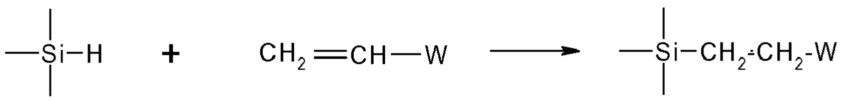

avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés.

A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule :

$$R_mR'SiO_{\frac{(3-m)}{2}} \quad (I)$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  o un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  o un groupement hydrocarboné cyclique insaturé comprenant de 5 à 8 atomes de carbone comme par exemple

un groupe cyclohexenyle.

De préférence R' est un groupement hydrocarboné aliphatique insaturé, de préférence un groupe vinyle.

Selon un mode de réalisation particulier, le polyorganosiloxane comprend également des unités de formule

$$R_nSiO_{\frac{(4-n)}{2}} \quad (II)$$

dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation. On peut citer par exemple les résines de type MQ ou MT portant elle-même des extrémités réactives insaturées $-CH=CH_2$.

Ces résines sont des polymères d'organosiloxanes réticulés.

La nomenclature des résines de silicone est connue sous le nom de "MDTQ", la résine étant décrite en fonction des différentes unités monomériques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle $SiO_{4/2}$ dans laquelle l'atome de silicium est lié à quatre atomes d'hydrogène eux mêmes liés au reste du polymère. Comme exemples de telles résine, on peut citer les résines de silicone MT telles que les poly(phényl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (1) et éventuellement (11) décrites précédemment.

Le composé Y comprend de préférence au moins deux groupes Si-H (groupes hydrogénosilanes) libres.

Le composé Y peut être avantageusement choisi parmi les organosiloxanes comprenant au moins une unité alkylhydrogénosiloxane de formule suivante :

$$R_pHSiO_{\frac{(3-p)}{2}} \quad (III)$$

dans laquelle :

R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogénosiloxanes peuvent comprendre en outre des unités de formule :

$$R_nSiO_{\frac{(4-n)}{2}} \quad (II)$$

telles que définies plus haut.

Le composé Y peut être une résine de silicone comprenant au moins un motif choisi parmi les motifs M, D, T, Q tels que définis ci-dessus et comprenant au moins un groupe Si-H telles que les poly(méthyl-hydridosilsesquioxane) commercialisées sous la référence SST-3MH1.1 par la société Gelest

De préférence, ces composés Y organosiloxanes comprennent de 0,5 à 2,5% en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule $(CH_3)_3SiO_{1/2}$.

Avantageusement, les organosiloxanes Y comprennent au moins deux unités alkylhydrogénosiloxane de formule $(H_3C)(H)SiO$ et comprennent éventuellement des unités $(H_3C)_2SiO$.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

a) Les polyesters à insaturation(s) éthylénique(s) :

Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.

b) les polyesters à groupes (méth)acrylate latéraux et/ou terminaux :

Il s'agit d'un groupe de polymères de type polyester obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la

demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,

- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule :

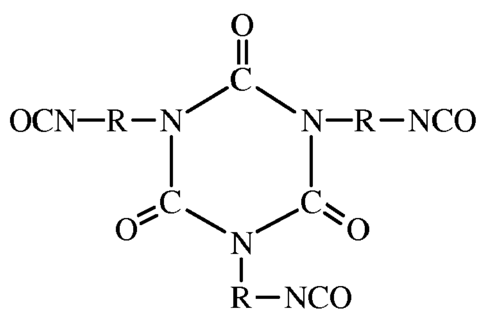

  résultant de la trimérisation de 3 molécules de diisocyanates OCN-R-CNO, où R est un radical hydrocarboné linéaire, ramifié ou cyclique comportant de 2 à 30 atomes de carbone ;
- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse

molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.

d) les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé. Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

e) les époxyacrylates obtenus par réaction entre

- au moins un diépoxyde choisi par exemple parmi :

  (i) l'éther diglycidylique de bisphénol A
  (ii) une résine diépoxy résultant de la réaction entre l'éther diglycidylique de bisphénol A et l'épichlorhydrine,
  (iii) une résine époxyester à extrémités $\alpha,\omega$-diépoxy résultant de la condensation d'un diacide carboxylique ayant de 3 à 50 atomes de carbone avec un excès stoechiométrique de (i) et/ou (ii),
  (iv) une résine époxyéther à extrémités $\alpha,\omega$-diépoxy résultant de la condensation d'un diol ayant de 3 à 50 atomes de carbone avec un excès stoechiométrique de (i) et/ou (ii),
  (v) les huiles naturelles ou synthétiques portant au moins 2 groupes époxyde, telles que l'huile de soja époxydée, l'huile de lin époxydée et l'huile de vernonia époxydée,
  (vi) un polycondensat phénol-formaldéhyde (résine Novolac®), dont les extrémités et/ou les groupes latéraux ont été époxydés,

  et
- un ou plusieurs acides carboxyliques ou polyacides carboxyliques comportant au moins une double liaison éthylénique en $\alpha,\beta$ du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.

De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

f) les poly(méth)acrylates d'(alkyle en $C_{1-50}$), ledit alkyle étant linéaire, ramifié ou cyclique, comportant au moins deux fonctions à double liaison éthylénique portées par les chaînes hydrocarbonées latérales et/ou terminales. De tels copolymères sont commercialisés par exemple sous les dénominations IRR® 375, OTA® 480 et EBECRYL®2047 par la société UCB.

g) les polyoléfines telles que le polybutène, le polyisobutylène.

h) les perfluoropolyéthers à groupes acrylate obtenus par estérification, par exemple par l'acide (méth)acrylique, de perfluoropolyéthers portant des groupes hydroxyle latéraux et/ou terminaux. De tels perfluoropolyéthers $\alpha,\omega$-diols sont décrits notamment dans EP-A-1057849 et sont commercialisés par la société AUSIMONT sous la dénomination FOMBLIN® Z DIOL.

i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique. Les dendrimères (du grec dendron = arbre) sont des

molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754). La société PERSTORP commercialise sous la dénomination BOLTORN® des polyesters hyperramifiés. On trouvera sous la dénomination COMBURST® de la société DENDRITECH des polyéthylèneamines hyperramifiées. Des poly (esteramide) hyperramifiés à extrémités hydroxyle sont commercialisés par la société DSM sous la dénomination HYBRANE®.
Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.
On peut donc utiliser ces polymères dendritiques et hyperramifiés en association avec un ou plusieurs des polymères et/ou oligomères a) à h) ci-dessus.

## 1a Composés réactifs additionnels

Selon un mode de réalisation, les compositions comprenant le composé X et/ou Y peuvent comprendre en outre un composé réactif additionnel tel que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotétraméthyl tétravinylsiloxane,
- des composés silazanes tels que l'hexaméthyldisilazane.

## 1b Catalyseur

La réaction d'hydrosilylation se fait avantageusement en présence d'un catalyseur qui peut être présent dans l'une ou l'autre des compositions comprenant le composé X et/ou le composé Y ou dans une composition séparée, le catalyseur étant de préférence à base de platine ou d'étain.
On peut citer par exemple les catalyseurs à base de platine déposé sur un support de gel de silice ou de poudre de charcoal (charbon), le chlorure de platine, les sels de platine et d'acides chloroplatiniques.
On utilise de préférence les acides chloroplatiniques sous forme hexahydrate ou anhydre, facilement dispersible dans les milieux organosiliconés.
On peut également citer les complexes de platine tels que ceux à base d'acide chloroplatinique hexahydrate et de divinyl tétraméthyldisiloxane
La catalyseur peut être présent dans l'une ou l'autre des compositions utiles dans la présente invention en une teneur allant 0,0001% à 20% en poids par rapport au poids total de la composition le comprenant.
On peut également introduire dans les compositions de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polyméthylvinylsiloxanes cycliques, et en particulier le tétravinyl tétraméthyl cyclotétrasiloxane, les alcools acétyléniques, de préférence volatiles, tels que le méthylisobutynol.
La présence de sels ioniques, tels que l'acétate de sodium, dans l'une et/ou l'autre des première et seconde compositions peut avoir une influence dans la vitesse de polymérisation des composés.
A titre d'exemple d'une combinaison de composés X et Y réagissant par hydrosilylation, on peut citer les références suivantes proposée par la société Dow Corning : DC 7-9800 Soft Skin Adhesive parts A & B, ainsi que les mélanges A et B suivants préparés par Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

De façon avantageuse, les composés X et Y sont choisis parmi les composés siliconés susceptibles de réagir par hydrosilylation ; en particulier le composé X est choisi parmi les polyorganosiloxanes comprenant des unités de formule (I) décrits ci-dessus et le composé Y est choisi parmi les organosiloxanes comprenant des unités alkylhydrogénosiloxanes de formule (III) décrits ci-dessus. Selon un mode de réalisation particulier, le composé X est un polydiméthylsiloxane à groupements vinyliques terminaux, et le composé Y est le méthylhydrogénosiloxane.

**2/ Composés X et Y susceptibles de réagir par condensation**

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, en particulier l'eau apportée par une source extérieure, par exemple par humidification préalable des cheveux (par exemple par un brumisateur).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule

$$R^9_sSiO_{(4-s)/2}, \qquad (IV)$$

dans laquelle $R^9$ représente indépendamment un radical choisi parmi les groupes alkyles comprenant de 1 à 6 atomes de carbone, le phényle, les groupes alkyl fluorés, et s est égal à 0, 1, 2 ou 3. De préférence, $R^9$ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule

$$(R^9{,}SiO_2)_f -\qquad (V)$$

dans laquelle $R^9$ est tel que décrit ci-dessus, de préférence $R^9$ est un radical méthyle, et f est notamment tel que le polymère présente avantageusement une viscosité à 25°C allant de 0,5 à 3000 Pa.s, de préférence allant de 5 à 150 Pa.s ; et/ou f est un nombre allant de 2 à 5000, plus particulièrement de 3 à 3000, et de préférence de 5 à 1000.

Ces composés X et Y polyorganosiloxanes comprennent au moins deux groupes trialcoxysilanes terminaux par molécule de polymère, lesdits groupes ayant la formule suivante

$$- ZSiR^1_x\,(OR)_{3-x},\qquad (VI)$$

dans laquelle
les radicaux R représentent indépendamment un groupe méthyle, éthyle, n-propyle, isopropyle, n-butyle, sec-butyle, isobutyle, de préférence un groupe méthyle ou éthyle, $R^1$ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone, de préférence de 2 à 18 atomes de carbone, (groupes alkylène), les combinaisons de radicaux hydrocarbonés divalents et de segments siloxanes de formule (IX) suivante :

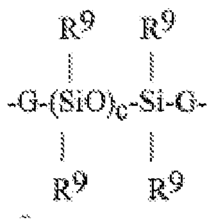

(IX)

$R^9$ étant tel que décrit plus haut, G est un radical hydrocarboné divalent ne comportant pas d'insaturation éthylénique et comprenant de 1 à 18 atomes de carbone, de préférence de 2 à 18 atomes de carbone, et c est un entier allant de 1 à 6.
Z et G peuvent être notamment choisis parmi les groupements alkylènes tels que le méthylène, l'éthylène, le propylène, le butylène, le pentylène, l'hexylène, les groupements arylène tels que le phenylène.

De préférence, Z est un groupe alkylène, et mieux ethylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule $CH_2=CH-SiR^9_2-$ ou de formule $R^6_3-Si-$, dans laquelle $R^9$ est tel que défini plus haut et chaque groupe $R^6$ est indépendamment choisi parmi les groupes $R^9$ ou vinyle. On peut citer comme exemples de tels groupements terminaux les groupes triméthoxysilane, triéthoxysilane, vinyldiméthoxysilane et vinylméthyloxyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier le polymère de formule

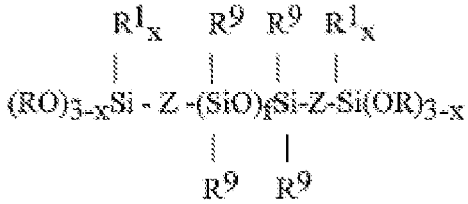

(VII)

dans laquelle R, $R^1$, $R^9$, Z, x et f sont tels que décrits plus haut.

Les composés X et/ou Y peuvent également comprendre un mélanges de polymère de formule (VII) ci-dessus avec des polymères de formule (VIII) suivante :

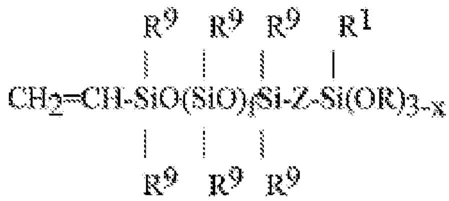

(VIII)

dans laquelle R, $R^1$, $R^9$, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40%, de préférence moins de 25% en nombre des chaînes terminales

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

Selon une variante l'un des deux composés réactifs X ou Y, est de nature silicone et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Les polymères organiques de nature vinylique ou (méth)acryliques, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes etc.

On peut citer par exemple les polymère (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux, on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant ellemême des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

## 2a Composé réactif additionnel

L'une des compositions utiles dans la présente invention peut comprendre en outre un composé réactif additionnel comprenant au moins deux groupes alcoxysilane ou silanol.

On peut citer par exemple une ou des particules organiques ou minérales comprenant à leur surface des groupements alcoxysilanes et/ou silanols, par exemple des charges traitées en surface par de tels groupes.

**2b Catalyseur**

La réaction de condensation peut se faire en présence d'un catalyseur à base de métal qui peut être présent dans l'une ou l'autre des compositions comprenant X et/ou Y ou dans une composition séparée. Le catalyseur utile dans ce type de réaction est de préférence un catalyseur à base de titane.

On peut citer notamment les catalyseurs à base de tetraalcoxytitane de formule

$$Ti(OR^2)_y(OR^3)_{4-y},$$

dans laquelle $R^2$ est choisi parmi les radicaux alkyle tertiaires tels que le tert butyle, le tert amyle et le 2,4-diméthyl-3-pentyl ; $R^3$ représente un radical alkyle comprenant de 1 à 6 atomes de carbone, de préférence un groupe methyle ethyle, n-propyle, isopropyle, n-butyle, sec-butyle, hexyle et y est un nombre allant de 3 à 4, mieux de 3,4 à 4.

Le catalyseur peut être présent dans l'une ou l'autre des compositions utiles dans la présente invention en une teneur allant 0,0001% à 20% en poids par rapport au poids total de la ou des compositions le contenant.

**2c Diluant**

Les compositions utiles comprenant X et/ou Y peuvent comprendre en outre une huile siliconée volatile (ou diluant) destinée à faire diminuer la viscosité de la composition.

Cette huile peut être choisie parmi les silicones linéaires à chaîne courte telles que l'hexaméthyldisiloxane, l'octaméthyltrisiloxane, les silicones cycliques telles que l'octaméthylcyclotétrasiloxane, la décaméthylclopentasiloxane et leurs mélanges.

Cette huile siliconée peut représenter de 5% à 95%, de préférence de 10 à 80% en poids par rapport au poids de chaque composition.

A titre d'exemple d'une combinaison de composés X et Y porteurs de groupements alcoxysilanes et réagissant par condensation, on peut citer les mélanges A' et B4 suivants préparés par la société Dow Corning :

**Mélange A' :**

| Ingrédient (Nom INCI) | N°CAS | Teneurs (%) | Fonction |
|---|---|---|---|
| Bis-Trimethoxysiloxyethyl Tetramethyldisiloxyethyl Dimethicone (*) | PMN87176 | 25-45 | Polymère |
| Silica Silylate | 68909-20-6 | 5-20 | Charge |
| Disiloxane | 107-46-0 | 30-70 | Solvant |
| (*) Il est à noter que les composés X et Y identiques sont réunis dans le mélange A'. | | | |

**Mélange B'**:

| Ingrédient (Nom INCI) | N°CAS | Teneurs (%) | Fonction |
|---|---|---|---|
| Disiloxane | 107-46-0 | 80-99 | Solvant |
| Tetra T Butyl Titanate | - | 1-20 | Catalyseur |

**3/ Réticulation en présence de peroxyde**

Cette réaction se fait de préférence par chauffage à une température supérieure ou égale à 50°C, de préférence supérieure ou égale à 80°C, allant jusqu'à 120°C.

Les composés X et Y, identiques ou différents, comprennent dans ce cas au moins deux groupements latéraux $-CH_3$ et/ou au moins deux chaînes latérales portant un groupement $-CH_3$.

Les composés X et Y sont de préférence siliconés et peuvent être choisis par exemple parmi les polydimethylsiloxanes linéaires non volatiles de haut poids moléculaire, ayant un degré de polymérisation supérieur à 6 présentant au moins deux groupements latéraux $-CH_3$ reliés à l'atome de silicium et/ou au moins deux chaînes latérales portant un groupement $-CH_3$. On peut citer par exemple les polymères décrits dans le Catalogue « Reactive Silicones » de la société Gelest Inc., Edition 2004, page 6, et notamment les copolymères (aussi appelés gommes) de vinylmethylsiloxane-dimethylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation ou la réaction de condensation ou bien encore la réaction de réticulation en présence d'un peroxyde entre les composés X et Y est accélérée par un apport de chaleur en élevant par exemple la température du système entre 25°C et 180°C. Le système réagira notamment sur la peau.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5% à 95%, de préférence de 10% à 90%, mieux encore de 20% à 80%.

De même, le pourcentage molaire de Y par rapport à l'ensemble des composés X et Y, c'est-à-dire le ratio Y/(X+Y) x 100, peut varier de 5% à 95%, de préférence de 10% à 90%, mieux encore de 20% à 80%.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,5 % à 95 % en poids par rapport au poids total de la composition, de préférence de 1 % à 90 % en poids, et mieux de 5 % à 80 % en poids.

Le composé Y peut représenter de 0,05 % à 95 % en poids par rapport au poids total de la composition, de préférence de 0,1 % à 90 % en poids et mieux de 0,2 % à 80 % en poids.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, si X et Y ne sont pas les mêmes, les composés X et Y peuvent être présents en un ratio X/Y molaire allant de 0,05 à 20 et mieux de 0,1 à 10.

## Les pigments

Au sens de la présente invention, on entend par pigment toute entité organique et/ou minérale dont la solubilité dans l'eau est inférieure à 0,01 % en poids à 20°C, de préférence inférieure à 0,0001 % en poids, et présentant une absorption entre 350 et 750 nm, de préférence une absorption avec un maximum.

Les pigments peuvent par exemple être choisis parmi les pigments minéraux, les pigments organiques, les laques, les pigments à effet spéciaux tels que notamment les nacres ou les paillettes, ou leurs mélanges.

Ces pigments peuvent être présents en une teneur allant de 0,01 % à 40 % en poids, de préférence de 0,1 % à 20 % par rapport au poids total de la composition.

Les pigments utiles dans la présente invention peuvent se présenter sous forme de poudre ou de pâte pigmentaire. Ils peuvent être enrobés ou non.

Par « nacres » ou pigments nacrés, il faut comprendre des particules colorées de toute forme, irisées ou non, notamment produites par certains mollusques dans leur coquille ou bien synthétisées et qui présentent un effet de couleur par interférence optique.

Les pigments ont plus particulièrement une taille moyenne en nombre allant de 2 nm à 5 mm, et mieux de 10 nm à 2 mm.

Il est à noter que la « taille moyenne» désigne la dimension maximale moyenne, qu'il est possible de mesurer entre deux points opposés d'une particule. La taille moyenne en nombre peut être déterminée par microscopie optique ou électronique.

Encore plus avantageusement, la taille moyenne en nombre du pigment est comprise entre 10 nm et 200 µm, de préférence entre 20 nm et 80 µm, et plus préférentiellement entre 30 nm et 50 µm.

Le pigment peut-être un pigment organique. Par pigment organique, on entend tout pigment qui répond à la définition de l'encyclopédie Ullmann dans le chapitre pigment organique.

Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes

codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

A titre d'exemple de pigments organiques, on peut aussi citer les pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R" : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R Pigment BLACK 7 (CI 77266).

Le pigment peut aussi être une laque. Par laque, on entend les colorants insolubilisés éventuellement adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les pigments organiques, on peut citer aussi le carmin de cochenille. On peut également citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 0 (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut être un pigment minéral.

Par pigment minéral, on entend tout pigment qui répond à la définition de l'encyclopédie Ullmann dans le chapitre pigment inorganique.

On peut citer, parmi les pigments minéraux utiles dans la présente invention, le dioxyde de titane traité ou non en surface, les oxydes de zirconium ou de cérium, ainsi que les oxydes de fer ou de chrome, le violet de manganèse, le bleu outremer, l'hydrate de chrome et le bleu ferrique. Les pigments minéraux suivants peuvent aussi être utilisés : $Ta_2O_5$, $Ti_3O_5$, $Ti_2O_3$, TiO, $ZrO_2$ en mélange avec $TiO_2$, $ZrO_2$, $Nb_2O_5$, $CeO_2$, ZnS.

On peut aussi envisager des pigments composites comprenant un noyau inorganique (choisi par exemple parmi ceux mentionnés auparavant) recouvert au moins partiellement d'un colorant dispersé dans au moins un liant tel que par exemple une résine époxy.

Le pigment peut aussi être un pigment à effets spéciaux.

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation, ...). Ils s'opposent par-là même aux pigments blancs ou colorés qui procurent une teinte uniforme opaque, semi-transparente ou transparente classique.

Il existe deux types de pigments à effets spéciaux, ceux à faible indice de réfraction tels que les pigments photochromes ou thermochromes, et ceux à plus fort indice de réfraction tels que les nacres ou les paillettes.

A titre de pigments à effets spéciaux, on peut citer les pigments nacrés tels que des pigments nacrés blancs par exemple le mica recouvert de titane, ou d'oxychlorure de bismuth, des pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre d'exemple, on peut citer les pigments Cellini commercialisée par Engelhard (Mica-$TiO_2$-laque), Prestige commercialisée par Eckart (Mica-$TiO_2$ Colorona commercialisée par Merck (Mica-$TiO_2$-$Fe_2O_3$).

En plus des nacres sur un support mica, on peut envisager les pigments multicouches basés sur des substrats

synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek).

Les pigments à effets spéciaux comprennent aussi les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

Les pigments peuvent être dispersées dans le produit grâce à un agent dispersant.

L'agent dispersant sert à protéger les particules dispersées contre leur agglomération ou floculation.

Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy 12-stéarique sont de préférence destinés à un milieu hydrocarboné ou fluoré, alors que les mélanges de diméthylsiloxane oxyéthylène/oxypropyléné sont de préférence destinés à un milieu siliconé.

Les pigments peuvent de même être traités en surface par un agent organique.

Plus particulièrement, les pigments préalablement traités en surface utiles dans le cadre de l'invention, sont des pigments ou des charges qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensioactifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou

les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :

- un traitement PEG-Silicone comme le traitement de surface AQ commercialisé par LCW;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

De préférence, s'ils sont enrobés, les pigments mis en oeuvre ne comprennent pas de revêtement hydrophobe.

Conformément à un mode de réalisation avantageux de l'invention, les pigments présents dans la composition sont des pigments colorés.

On entend par pigments colorés des pigments autres que des pigments blancs.

Plus particulièrement, ces pigments lorsqu'ils sont déposés de manière jointive sur un support noir, c'est-à-dire lorsque les particules sont au contact les unes des autres, présentent une différence de coloration avec l'étalon blanc de référence du colorimètre qui est supérieure ou égale à 2, et de préférence supérieure à 10.

La différence de coloration avec l'étalon blanc de référence du colorimètre est évaluée par l'écart de couleur

$$\text{DE} = \sqrt{(L_i * - L_0 *)^2 + (a_i * - a_0 *)^2 + (b_i * - b_0 *)^2}$$

$L_i$*, $a_i$*, $b_i$* et $L_0$*, $a_0$*, $b_0$* étant respectivement les coordonnées colorimétriques dans le système La*b* des pigments colorés et de l'étalon blanc de référence du colorimètre

**Les agents texturisants**

La composition appliquée sur les fibres capillaires peut en outre comprendre un ou plusieurs agents texturisants (charges), différents des pigments présents dans la composition.
On entend par agents texturisants, des particules minérales ou de synthèse, lamellaires ou non lamellaires, insolubles dans l'eau.
A titre d'exemple, ces agents texturisants peuvent être du carbonate de calcium colloïdal qui peut être traité ou non par de l'acide stéarique ou du stéarate, de la silice telle que les silices pyrogénées les silices précipitées, les silices traitées pour les rendre hydrophobes, du quartz moulu, de l'alumine, de l'hydroxyde d'aluminium, de la terre de diatomée.
On peut aussi citer le talc, le mica, le kaolin, les poudres de polyamide (Nylon@) (Orgasol de chez Atochem), les poudres de polyéthylène, les poudres de polymères de tétrafluoroéthylène (TéflonB), l'amidon, des microsphères polymériques telles que celles de chlorure de polyvinylidène/acrylonitrile comme l'Expancel (Nobel Industrie), de copolymères d'acide acrylique (PolytrapB de la société Dow Corning).
Les silices synthétiques dont la surface est modifiée par des composés siliconés pour les rendre hydrophobe en surface sont particulièrement préférées. Ces agents se différentient les uns des autres par leurs propriétés de surface, les composés de silicone utilisés pour traiter la silice, et la façon dont le traitement en surface est réalisé.
De tels agents permettent de modifier la viscosité de la formulation obtenue à partir des composés X et/ou Y et/ou de modifier les propriétés du matériau obtenu.
On préfère à titre d'agent texturisant la silice, le carbonate de calcium, les agents à base de résine.
A titre d'exemple, on peut citer les silices traitées Cab-0-Sil@TS-530, Aerosil@R8200 et Wacker HDX H2000.
Les agents texturisants peuvent être présents à raison de 0 à 48 % en poids, par rapport au poids total de la composition, de préférence 0,01 à 30 % en poids, et mieux de 0,02 % à 20 % en poids.

**Solvants organiques**

La composition mise en oeuvre dans le cadre de l'invention peut comprendre au moins un solvant organique.
Par solvant organique, on entend une substance organique liquide à la température de 25 °C et à la pression atmosphérique (760 mm de Hg) capable de dissoudre une autre substance sans la modifier chimiquement.
Il est à noter que le ou les solvants organiques sont distincts des composés X et Y utiles dans le cadre de l'invention.
Le ou les solvants organiques sont par exemple choisis parmi les alcools aromatiques tels que l'alcool benzylique, le phénoxyéthanol, l'alcool phényléthylique ; les alcools gras liquides, notamment en $C_{10}$-$C_{30}$ ; les alcanols en $C_1$-$C_6$ tels que l'éthanol, l'isopropanol, le n-propanol, le butanol, le n-pentanol, le 1,2-propanediol, le 1,3-propanediol, le 1-méthoxy 2-propanol, le 1-éthoxy 2-propanediol, les 1,3 et 1,4-butanediol, le 1,2-hexanediol ; les polyols et éthers de polyols possédant une fonction - OH libre tels que le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol, le glycérol, le glycol, le dipropylène glycol, le butylène glycol, le butyle diglycol ; les silicones volatiles telles que les silicones linéaires à chaîne courte telles que l'hexaméthyldisiloxane et l'octaméthyltrisiloxane, les silicones cycliques telles que l'octaméthylcyclotétrasiloxane, la décaméthylcyclopentasiloxane, la dodécaméthylcyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire; les polydiméthylsiloxanes modifiées liquides ; les huiles minérales, organiques ou végétales ; les alcanes et plus particulièrement les alcanes linéaires ou ramifiés, de $C_5$ à $C_{20}$ ; les acides gras liquides ; les esters gras liquides et plus particulièrement les benzoates ou les salicylates d'alcool gras liquides.
Le ou les solvants organiques sont de préférence choisis parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes linéaires ou ramifiés, en $C_5$-$C_{20}$ comme l'isododécane, l'isohexadécane, les composés isoparaffiniques du type des produits commercialisés sous la dénomination Isopar E, l'acétone, la méthyléthylcétone, les esters d'acides en $C_1$-$C_{20}$ liquides et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras liquides en $C_{10}$-$C_{30}$ tels que l'alcool oléique, les esters d'alcools gras ou d'acides gras liquides tels que les benzoates d'alcool gras en $C_{10}$-$C_{30}$ et leurs mélanges ; l'isononanoate d'isononyle, le malate d'isostéaryle, le tétra-isostéarate de pentaérythrityle, le trimélate de tridécyle ; l'huile de polybutène ; le mélange cyclopentasiloxane (14,7 % en poids) / polydiméthylsiloxane dihydroxylé en positions $\alpha$ et w (85,3 % en poids), ou leurs mélanges.
Selon un mode de réalisation préféré, le ou les solvants organiques sont choisis parmi les silicones telles que

les polydiméthylsiloxanes liquides et les polydiméthylsiloxanes modifiées liquides, leur viscosité à 25 °C étant comprise entre 0,1 cst et 1 000 000 cst, et plus préférentiellement entre 1 cst et 30 000 cst.

On citera de préférence les huiles suivantes :

- le mélange de polydiméthylsiloxane alpha-omega-dihydroxylé / cyclopentadiméthylsiloxane (14,7 / 85,3) commercialisé par Dow Corning sous le nom de DC 1501 Fluid ;
- le mélange de polydiméthylsiloxane alpha-omega-dihydroxylé / polydiméthylsiloxane commercialisé par Dow Corning sous le nom de DC 1503 Fluid ;
- le mélange de diméthicone / cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
- et les mélanges respectifs de ces huiles.

Ces solvants organiques également peuvent servir de diluant pour les réactions entre X et Y.

Le ou les solvants organiques de la composition représentent généralement de 0,01 à 99 %, de préférence de 50 à 99 % en poids par rapport au poids total de la composition.

La composition de l'invention peut contenir, outre le ou les solvants organiques, de l'eau dans une proportion allant de 1 à 99 % et de préférence de 1 à 50 % par rapport au poids total de la composition.

La composition de l'invention peut aussi être anhydre, c'est-à-dire contenant moins de 1 % en poids d'eau par rapport au poids total de la composition.

Ces compositions peuvent se présenter sous des formes diverses, telles que des lotions, des aérosols, des mousses et être appliquées sous forme de shampooing ou d'après-shampooing.

Dans le cas des aérosols, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

On pourra aussi utiliser des aérosols à poche(s) contenant une ou plusieurs poches.

Pour l'application des produits de manière générale, on pourra utiliser tout dispositif comportant des zones de stockage multiples et un système de délivrance avec un ou plusieurs orifices permettant le mélange des produits à la sortie de ce dispositif.

## Mode d'application

Le ou les composés X, Y, le ou les pigments, peuvent être appliqués sur les fibres capillaires à partir de plusieurs compositions contenant le ou les composés X, le ou les composés Y, le ou les pigments, seuls ou en mélange, ou à partir d'une seule composition contenant le ou les composés X, le ou les composés Y, le ou les pigments.

Ainsi, selon un premier mode de réalisation particulier de l'invention, on applique sur les cheveux une composition (A) comprenant le ou les composés X, le ou les composés Y, le ou les pigments.

Cette composition (A) peut donc être obtenue par mélange extemporané avant l'application, soit des ingrédients pris séparément, soit de diverses compositions comprenant chacune une partie des ingrédients constitutifs de la composition (A).

De préférence, la composition (A) comprend au moins un solvant organique. On pourra se reporter à la partie correspondante de la description pour ce qui concerne la nature du solvant.

Selon un autre mode de réalisation particulier de l'invention, on applique sur les cheveux une composition (B) comprenant le ou les pigments, et une composition (C) comprenant le ou les composés X et le ou les composés Y, l'ordre d'application des compositions (B) et (C) étant indifférent.

Selon un autre mode de réalisation particulier de l'invention, on applique sur les cheveux une composition (B) comprenant le ou les pigments, une composition (D) comprenant le ou les composés X et une composition (E) comprenant le ou les composés Y, l'ordre d'application des compositions (B), (D) et (E) étant indifférent.

Selon un autre mode de réalisation particulier de l'invention, on applique sur les cheveux une composition (F) comprenant le ou les composés X et le ou les pigments et une composition (E) comprenant le ou les composés Y, l'ordre d'application des compositions (F) et (E) étant indifférent.

Selon un autre mode de réalisation particulier de l'invention, on applique sur les cheveux une composition (D) comprenant le ou les composés X et une composition (G) comprenant le ou les composés Y et le ou les pigments se trouvant dans la composition (D) et/ou la composition (G), l'ordre d'application des compositions (D) et (G) étant indifférent.

Selon un mode de réalisation préféré de l'invention, la composition comprenant le ou les pigments est appliquée avant la ou les compositions comprenant le ou les composés X et / ou le ou les composés Y.

Selon un autre mode de réalisation particulier de l'invention, on applique sur les cheveux au moins un catalyseur et/ou au moins un peroxyde, pour activer la réaction entre le ou les composés X et le ou les composés Y. Dans ce cas, le ou les catalyseurs et/ou le(s) peroxyde(s) sont présents dans une ou dans plusieurs des compositions appliquées déjà décrites sur les cheveux ou encore dans une ou deux compositions supplémentaires, auquel cas l'ordre d'application des différentes compositions sur les fibres est indifférent. Il est précisé que dans le cas de ce mode de réalisation, le catalyseur et/ou le peroxyde ne se trouve(nt) pas dans la composition (C) détaillée auparavant, si cette composition est destinée à être stockée avant l'application.

Les catalyseurs et peroxydes ont été décrits auparavant et l'on pourra se reporter à la description correspondante.

Selon un autre mode de réalisation particulier de l'invention, on applique sur les fibres capillaires au moins un composé réactif additionnel tel que décrit précédemment.

Par exemple, le ou les composés réactifs additionnels peuvent être présents dans une ou dans plusieurs des compositions déjà décrites appliquées sur les fibres capillaires ou dans une composition supplémentaire, auquel cas l'ordre d'application des différentes compositions sur les fibres est indifférent.

Dans une variante préférée de l'invention, au moins l'une des compositions contient au moins un solvant organique.

Les différentes compositions mises en oeuvre dans le procédé conforme à l'invention peuvent être appliquées sur des cheveux secs ou humides.

Un séchage et/ou un rinçage intermédiaire peut être réalisé entre chaque application.

Le dépôt ainsi formé présente l'avantage d'avoir une faible solubilité attendue. En outre, il possède une bonne affinité pour la surface des fibres kératiniques, ce qui garantit une meilleure rémanence de l'ensemble du dépôt.

Lorsque les composés X et Y sont appliqués séparément, le dépôt en couches obtenu peut aussi être avantageux pour conserver les propriétés cosmétiques ou optiques du composé qui constitue la partie supérieure du dépôt.

Selon les mêmes procédés, il est possible de réaliser des superpositions multiples de couches de composés X et Y, en alternance ou non, pour atteindre le type de dépôt souhaité (en termes de nature chimique, résistance mécanique, épaisseur, aspect, toucher...).

La présente invention a également pour objet l'utilisation pour le traitement des fibres capillaires :

- d'au moins un composé X et d'au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- d'au moins un pigment ; et éventuellement,
- d'au moins un solvant organique,

le ou les composés X, le ou les composés Y, le ou les pigments étant tels que définis précédemment.

En particulier, la présente invention a pour objet l'utilisation pour le gainage rémanent des fibres capillaires :

- d'au moins un composé X et d'au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- d'au moins un pigment ; et éventuellement
- d'au moins un solvant organique,

le ou les composés X, le ou les composés Y, le ou les pigments étant tels que définis précédemment.

L'invention est illustrée plus en détails par les exemples décrits ci-après.

Sauf indication contraire, dans ce qui va suivre, les pourcentages sont exprimés en poids.

**EXEMPLE 1**

Cet exemple est réalisé à partir des mélanges suivants préparés par la société Dow Corning :

**Mélange A'**:

| Ingrédient (Nom INCI) | N°CAS | Teneurs (%) | Fonction |
|---|---|---|---|
| Bis-Trimethoxysiloxyethyl | PMN87176 | 25-45 | Polymère |

(suite)

| Ingrédient (Nom INCI) | N°CAS | Teneurs (%) | Fonction |
|---|---|---|---|
| Tetramethyldisiloxyethyl Dimethicone | | | |
| Silica Silylate | 68909-20-6 | 5-20 | Charge |
| Disiloxane | 107-46-0 | 30-70 | Solvant |

**Mélange B' :**

| Ingrédient (Nom INCI) | N°CAS | Teneurs (%) | Fonction |
|---|---|---|---|
| Disiloxane | 107-46-0 | 80-99 | Solvant |
| Tetra T Butyl Titanate | - | 1-20 | Catalyseur |

On prépare les compositions suivantes :

**Composition 1**

| | |
|---|---|
| Cyclopentadiméthylsiloxane commercialisé par Dow Corning sous le nom de DC245 Fluid | 73,36 g |
| Mélange A' | 15,15 g |
| Nacre mica enrobé d'oxyde de fer brun commercialisée sous la dénomination Prestige Bronze par Eckart | 10 g |

**Composition 2**

| | |
|---|---|
| Mélange B' | 1,51 g |

On mélange de façon extemporanée les compositions 1 et 2 pour obtenir 100g d'un mélange.
0,5g de ce mélange est appliqué sur une mèche de 1g de cheveux propres et humides.
Après une heure de pose, la mèche est séchée au sèche-cheveux pendant deux minutes.
On obtient une mèche dont les cheveux sont individuellement colorés.

**EXEMPLE 2**

Dans cet exemple, on utilise les mélanges A et B suivants préparés par la société Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |

(suite)

| Ingrédient (Nom INCI) | N°CAS | Teneurs (%) | Fonction |
|---|---|---|---|
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

On prépare les deux compositions suivantes :

**Composition 1**

| | |
|---|---|
| Cyclopentadiméthylsiloxane commercialisé par Dow Corning sous le nom de DC245 Fluid | 70 % |
| Mélange A | 10 % |
| Nacre mica enrobé d'oxyde de fer brun commercialisée sous la dénomination Prestige Bronze par Eckart | 20 % |

**Composition 2**

| | |
|---|---|
| Cyclopentadiméthylsiloxane commercialisé par Dow Corning sous le nom de DC245 Fluid | 90 % |
| Mélange B | 10 % |

On mélange de façon extemporanée les compositions 1 et 2 ci-dessus en proportion 50/50.
0,5g de ce mélange résultant est appliqué sur une mèche de 1 g de cheveux propres et humides.
Après une heure de pose, la mèche est séchée au sèche-cheveux pendant deux minutes.
On obtient une mèche dont les cheveux sont individuellement colorés.

**EXEMPLE 3**

Dans cet exemple, on utilise les mélange A' et B' de l'exemple 1.
On prépare les compositions suivantes :

**Composition 1**

| | |
|---|---|
| Mélange A' | 98 % |
| Paillettes Crystallina 322 008X008 (Meadowbrook) | 2 % |

**Composition 2**

| | |
|---|---|
| Mélange B' | 100 % |

Les compositions 1 et 2 sont mélangées avec le rapport 10/1 avant l'application sur cheveux humides.
Les cheveux sont séchés à l'air libre pendant 30 minutes puis mis sous casque pendant 30 minutes.
On obtient des coiffures à effet paillettes, résistant à plusieurs shampooings.

**EXEMPLE 4**

On utilise les mélanges A et B de l'exemple 2
On prépare les compositions suivantes :

**Composition 1**

| | |
|---|---|
| Mélange A | 49 % |
| Cyclopentadiméthylsiloxane commercialisé par Dow Corning sous le nom de DC245 Fluid | 49 % |
| Paillettes Aluminium Pale Gold 3E 008X008 (Meadowbrook) | 2 % |

**Composition 2**

| | |
|---|---|
| Mélange B | 50 % |
| Cyclopentadiméthylsiloxane commercialisé par Dow Corning sous le nom de DC245 Fluid | 50 % |

Les compositions 1 et 2 sont mélangées avec le rapport 1/1 avant l'application sur cheveux secs ou humides.
Les cheveux sont séchés à l'air libre pendant 30 minutes puis mis sous casque pendant 30 minutes.
On obtient des coiffures à effet paillettes, résistant à plusieurs shampooings.

**Revendications**

1. Procédé de traitement de fibres capillaires, dans lequel on applique sur les fibres une composition comprenant au moins un pigment, au moins un composé X, au moins un composé Y, au moins un des composés X ou Y étant siliconé, lesdits composés X et Y étant susceptibles de réagir ensemble par une réaction d'hydrosilylation, ou par une réaction de condensation ou par une réaction de réticulation en présence d'un peroxyde, lorsqu'ils sont mis en contact les uns avec les autres.

2. Procédé selon la revendication précédente, **caractérisé en ce que** l'on applique simultanément ou séparément au moins un catalyseur.

3. Procédé selon la revendication précédente, **caractérisé en ce que** la composition comprend au moins un solvant organique.

4. Procédé selon l'une des revendications précédentes, **caractérisé en ce que** les composés X et Y sont susceptibles de réagir par hydrosilylation.

5. Procédé selon l'une quelconque des revendications 1 à 4, **caractérisé en ce que** le composé X est choisi parmi les composés siliconés comprenant au moins deux groupements aliphatiques insaturés.

**6.** Procédé selon la revendication précédente, **caractérisé en ce que** le composé X est un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal).

**7.** Procédé selon l'une quelconque des revendications 1 à 5, **caractérisé en ce que** le composé X est choisi parmi les polyorganosiloxanes comprenant au moins deux groupements aliphatiques insaturés liés chacun à un atome de silicium

**8.** Procédé selon l'une quelconque des revendications 1 à 7, **caractérisé en ce que** le composé X est choisi parmi les polyorganosiloxanes comprenant des unités siloxanes de formule :

$$R_mR'SiO_{\frac{(3-m)}{2}} \quad (I)$$

dans laquelle :

- R représente un groupe hydrocarboné monovalent, linéaire ou cyclique, comprenant de 1 à 30 atomes de carbone,
- m est égal à 1 ou 2 et
- R' représente un groupement hydrocarboné aliphatique insaturé comprenant de 2 à 10, de préférence de 3 à 5 atomes de carbone ou un groupement hydrocarboné cyclique insaturé comprenant de 5 à 8 atomes de carbone.

**9.** Procédé selon l'une quelconque la revendication précédente, **caractérisé en ce que** le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

**10.** Procédé selon l'une quelconque des revendications 8 ou 9, **caractérisé en ce que** R représente un radical alkyle comprenant de 1 à 10 atomes de carbone ou encore un groupement phényle et R' est choisi parmi les groupements vinyle.

**11.** Procédé selon l'une quelconque des revendications 1 à 10, **caractérisé en ce que** les polyorganosiloxanes comprennent en outre des unités de formule

$$R_nSiO_{\frac{(4-n)}{2}} \quad (II)$$

dans laquelle R est un groupe tel que défini dans la revendication 8, est n est égal à 1, 2 ou 3.

**12.** Procédé selon l'une des revendications 1 à 4, **caractérisé en ce que** le composé X est choisi parmi les oligomères ou polymères organiques, les oligomères ou polymères hybrides organique/silicone, lesdits oligomères ou polymères portant au moins 2 groupements aliphatiques insaturés réactifs et leurs mélanges.

**13.** Procédé selon l'une quelconque des revendications 1 à 12, **caractérisé en ce que** le composé Y est choisi parmi les organosiloxanes comprenant au moins deux groupes Si-H libres

**14.** Procédé selon la revendication 13, **caractérisé en ce que** le composé Y est choisi parmi les organosiloxanes comprenant au moins une unité alkylhydrogènosiloxanes de formule suivante :

$$R_pHSiO_{\frac{(3-p)}{2}} \quad (III)$$

dans laquelle R représente un groupe hydrocarboné monovalent, linéaire ou cyclique, comprenant de 1 à 30 atomes de carbone ou un groupe phényle, et p est égal à 1 ou 2.

**15.** Procédé selon la revendication précédente, dans lequel le composé Y est tel que les radicaux R représentent un groupement alkyle en C1-C10, de préférence méthyle.

**16.** Procédé selon l'une quelconque des revendications 13 à 15, dans lequel les organosiloxanes Y comprennent au moins deux unités alkylhydrogénosiloxane de formule $H_3CHSiO$ et comprennent éventuellement des unités $(H_3C)_2SiO$.

**17.** Procédé selon l'une quelconque des revendications 1 à 16, **caractérisé en ce que** le catalyseur est un catalyseur à base de platine ou d'étain.

**18.** Procédé selon la revendication 17, **caractérisé en ce que** le catalyseur représente de 0,0001 % à 20% en poids par rapport au poids total de la composition.

**19.** Procédé selon l'une quelconque des revendications 1 à 18, **caractérisé en ce que** le composé X est un polydiméthylsiloxane à groupements vinyliques terminaux et le composé Y est le méthylhydrogénosiloxane.

**20.** Procédé selon l'une des revendications 1 à 3, **caractérisé en ce que** le composé X et le composé Y sont susceptibles de réagir par condensation.

**21.** Procédé selon la revendication 20, **caractérisé en ce que** les composés X et/ou Y, identiques ou différents, sont des composés siliconés dont la chaîne principale comprend au moins deux groupes alcoxysilane et/ou au moins deux groupes silanol (Si-OH), latéraux et/ou en bout de chaîne.

**22.** Procédé selon l'une des revendications 20 ou 21, **caractérisé en ce que** les composés X et/ou Y, identiques ou différents comprennent de façon majoritaire des unités de formule

$$R^9{}_sSiO_{(4-s)/2}, \quad (IV)$$

dans laquelle les groupements $R^9$ représentent indépendamment un radical choisi parmi les groupes alkyles comprenant de 1 à 6 atomes de carbone, le phényle, les groupes alkyl fluorés, et s est égal à 0, 1, 2 ou 3.

**23.** Procédé selon la revendication 22, **caractérisé en ce que** les composés X et/ou Y, identiques ou différents comprennent des unités de formule

$$(R^9{}_2SiO_2)_f - \quad (V)$$

dans laquelle $R^9$ est tel que défini dans la revendication 22, et
f est notamment tel que le polymère présente une viscosité à 25°C allant de 0,5 à 3000 Pa.s, et/ou
f est notamment un nombre allant de 2 à 5000.

**24.** Procédé selon l'une quelconque des revendications 20 à 23, **caractérisé en ce que** les polyorganosiloxanes comprennent au moins 2 groupes trialcoxysilane terminaux par molécule de polymère, lesdits groupes ayant la formule suivante

$$ZSiR^1{}_x(OR)_{3-x} \quad (VI)$$

dans laquelle

- les radicaux R représentent indépendamment un groupe méthyle, éthyle, n-propyle, isopropyle, n-butyle, sec-

butyle, isobutyle,
- $R^1$ est un groupe méthyle ou éthyle,
- x est égal à 0 ou 1, de préférence x est égal à 0, et
- Z est choisi parmi les groupes hydrocarbonés divalents ne comportant pas d'insaturation éthylénique et comprenant de 1 à 18 atomes de carbone, les combinaisons de radicaux hydrocarbonés divalents et de segments siloxanes de formule (IX) :

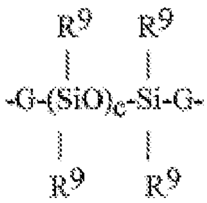

$R^9$ étant tel que défini dans la revendication 23, G est un radical hydrocarboné divalent ne comportant pas d'insaturation éthylénique et comprenant de 1 à 18 atomes de carbone et c est un entier allant de 1 à 6.

**25.** Procédé selon l'une quelconque des revendications 20 à 24, **caractérisé en ce que** les polyorganosiloxanes sont choisis parmi les polymères de formule :

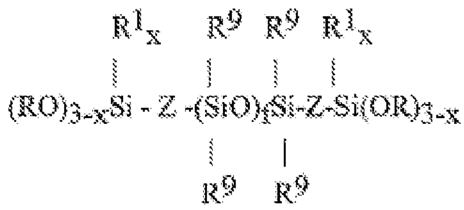

(VII)

dans laquelle R, $R^1$, $R^9$, Z, x et f sont tels que définis dans l'une des revendications 22 à 24.

**26.** Procédé selon la revendication 20, **caractérisé en ce que** le composé X est choisi parmi les oligomères ou polymères organiques ou les oligomères et polymères hybrides organique/silicone, lesdits polymères ou oligomères comprenant au moins deux groupements alcoxysilanes et le composé Y est choisi parmi les polyorganosiloxanes.

**27.** Procédé selon l'une des revendications 20 à 26, **caractérisé en ce que** le catalyseur est à base de titane.

**28.** Procédé selon la revendication précédente, **caractérisé en ce que** le catalyseur est présent en une teneur allant de 0,0001 % à 20% en poids par rapport au poids total de la composition.

**29.** Procédé selon l'une quelconque des revendications 20 à 29 dans lequel les composés X et Y représentent un mélange de polydiméthylsiloxanes à groupements méthoxysilanes.

**30.** Procédé selon l'une quelconque des revendications 1 à 3, **caractérisé en ce que** les composés X et Y sont susceptibles de réagir par réticulation en présence de peroxyde.

**31.** Procédé selon l'une quelconque des revendications 1 à 30, **caractérisé en ce que** la composition comprend un agent texturisant.

**32.** Procédé selon l'une quelconque des revendications 1 à 31, **caractérisé en ce que** le composé X présente une masse moléculaire moyenne en poids (Mw) allant de 150 à 1 000 000, de préférence de 200 à 800 000, de préférence encore de 200 à 250 000.

**33.** Procédé selon l'une des revendications précédentes, **caractérisé en ce que** le composé Y présente une masse moléculaire en poids (Mw) allant de 200 à 1 000 000, de préférence de 300 à 800 000, de préférence encore de 500 à 250 000.

**34.** Procédé selon l'une quelconque des revendications 1 à 33, **caractérisé en ce que** le composé X représente de 0,5 % à 95 % en poids par rapport au poids total de la composition.

**35.** Procédé selon l'une quelconque des revendications 1 à 34, **caractérisé en ce que** le composé Y représente de 0,05 % à 95 % en poids par rapport au poids total de la composition.

**36.** Procédé selon l'une quelconque des revendications 1 à 35, **caractérisé en ce que** les composés X et Y sont présents dans la composition en un ratio molaire X/Y allant de 0,05 à 20.

**37.** Procédé selon l'une quelconque des revendications 1 à 36, **caractérisé en ce que** le pigment est choisi parmi les pigments organiques.

**38.** Procédé selon l'une quelconque des revendications 1 à 37, **caractérisé en ce que** le pigment est choisi parmi les pigments minéraux.

**39.** Procédé selon l'une quelconque des revendications 1 à 38, **caractérisé en ce que** le pigment est choisi parmi les pigments composites.

**40.** Procédé selon l'une quelconque des revendications 1 à 39, **caractérisé en ce que** le pigment est choisi parmi les pigments à effets spéciaux.

**41.** Procédé selon l'une quelconque des revendications 1 à 41, **caractérisé en ce que** le pigment est présent en quantité varie de 0,01 à 40 % en poids par rapport au poids de la composition.

**42.** Procédé selon l'une quelconque des revendications 1 à 41, **caractérisé en ce que** l'on applique une composition obtenue par mélange extemporané d'une composition (B) comprenant le ou les pigments, avec une composition (C) comprenant le ou les composés X et le ou les composés Y, l'ordre d'application des compositions (B) et (C) étant indifférent

**43.** Procédé selon l'une quelconque des revendications 1 à 41, **caractérisé en ce que** l'on applique une composition obtenue par mélange extemporané d'une composition (B) comprenant le ou les pigments, une composition (D) comprenant le ou les composés X et une composition (E) comprenant le ou les composés Y, l'ordre d'application des compositions (B), (D) et (E) étant indifférent.

**44.** Procédé selon l'une quelconque des revendications 1 à 41, **caractérisé en ce que** l'on applique une composition obtenue par mélange extemporané d'une composition (F) comprenant le ou les composés X et le ou les pigments et une composition (E) comprenant le ou les composés Y, l'ordre d'application des compositions (F) et (E) étant indifférent.

**45.** Procédé selon l'une quelconque des revendications 1 à 41, **caractérisé en ce que** l'on applique une composition obtenue par mélange extemporané d'une composition (D) comprenant le ou les composés X et une composition (G) comprenant le ou les composés Y et le ou les pigments, l'ordre d'application des compositions (D) et (G) étant indifférent.

**46.** Procédé selon l'une des revendications 42 à 45, **caractérisé en ce que** le catalyseur ou le peroxyde est présent dans l'une et ou l'autre des compositions (B) à (G) à la condition que les composés X, Y et le catalyseur lorsqu'il est présent ou le peroxyde ne sont pas présents simultanément dans la même composition.

**47.** Utilisation pour le traitement des fibres capillaires :

- d'au moins un composé X et d'au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- d'au moins un pigment ; et éventuellement,
- d'au moins un solvant organique,

le ou les composés X, le ou les composés Y, le ou les pigments étant tels que définis dans l'une quelconque des revendications 1 à 41.

**48.** Utilisation pour le gainage rémanent des fibres capillaires :

- d'au moins un composé X et d'au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- d'au moins un pigment ; et éventuellement
- d'au moins un solvant organique,

le ou les composés X, le ou les composés Y, le ou les pigments étant tels que définis dans l'une quelconque des revendications 1 à 41.

Office européen des brevets

# RAPPORT DE RECHERCHE EUROPEENNE

Numéro de la demande
EP 07 12 3588

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 101 487 A (DOW CORNING TORAY SILICONE [JP]) 23 mai 2001 (2001-05-23)<br>* revendication 9; exemples * | 1-48 | INV.<br>A61K8/89<br>A61Q5/00<br>A61Q5/12<br>A61Q5/10 |
| X | US 2003/203978 A1 (O'BRIEN MICHAEL JOSEPH [US] ET AL) 30 octobre 2003 (2003-10-30)<br>* alinéas [0002], [0014]; revendications 24-27,43-46,48,62-65,67; exemples * | 1-48 | |
| X | DATABASE WPI Week 200010<br>Thomson Scientific, London, GB; AN 2000-111589<br>XP002445182<br>& JP 11 349450 A (SHISEIDO CO LTD) 21 décembre 1999 (1999-12-21)<br>* abrégé * | 1-48 | |
| X | EP 0 865 787 A1 (DOW CORNING SA [FR]) 23 septembre 1998 (1998-09-23)<br>* le document en entier * | 1-48 | |
| D,X | GB 2 407 496 A (DOW CORNING [US]) 4 mai 2005 (2005-05-04)<br>* le document en entier * | 1-48 | DOMAINES TECHNIQUES RECHERCHES (IPC)<br>A61K<br>A61Q |
| P,X | WO 2007/071885 A (OREAL [FR]; JAEGER LEZER NATHALIE [FR]) 28 juin 2007 (2007-06-28)<br>* le document en entier * | 1-48 | |

~~Le présent rapport a été établi pour toutes les revendications~~

4

| Lieu de la recherche | Date d'achèvement de la recherche | Examinateur |
|---|---|---|
| Munich | 20 mars 2008 | Mitchell, Gemma |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................................................
& : membre de la même famille, document correspondant

EPO FORM 1503 03.82 (P04C02)

Office européen
des brevets

Numéro de la demande
EP 07 12 3588

## REVENDICATIONS DONNANT LIEU AU PAIEMENT DE TAXES

La présente demande de brevet européen comportait lors de son dépôt plus de dix revendications

☐ Une partie seulement des taxes de revendication ayant été acquittée dans les délais prescrits, le présent rapport de recherche européenne a été établi pour les dix premières revendications ainsi que pour celles pour lesquelles les taxes de revendication ont été acquittées, à savoir les revendication(s):

☐ Aucune taxe de revendication n'ayant été acquittée dans les délais prescrits, le présent rapport de recherche européenne a été établi pour les dix premières revendications.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

voir feuille supplémentaire B

☐ Toutes les nouvelles taxes de recherche ayant été acquittées dans les délais impartis, le présent rapport de recherche européenne a été établi pour toutes les revendications.

☐ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☒ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

see annex

☐ Le present rapport supplémentaire de recherche européenne a été établi pour les parties de la demande de brevet européen qui se rapportent a l'invention mentionée en premier lieu dans le revendications (Règle 164 (1) CBE)

Office européen des brevets

## ABSENCE D'UNITÉ D'INVENTION FEUILLE SUPPLÉMENTAIRE B

Numéro de la demande
EP 07 12 3588

La division de la recherche estime que la présente demande de brevet européen ne satisfait pas à l'exigence relative à l'unité d'invention et concerne plusieurs inventions ou pluralités d'inventions, à savoir :

1. revendications: 1-3(part), 4, 5-19(part), 31-48(part)

Un procédé de traitement de fibres capillaires, dans lequel on applique sur les fibres une composition comprenant au moins un pigment, au moins un composé X, au moins un composé Y, au moins un des composés X et Y étant siliconé, lesdites composé X et Y étant susceptibles de réagir ensemble par (1) une réaction d'hydrosilylation, lorsqu'ils sont mis en contact les uns avec les autres; utilsation pour le traitement des fibres capillaires; et utilisation pour le gainage rémanent des fibres capillaires.

---

2. revendications: 1-2(part), 5-19(part), 20-29, 31-48(part)

Un procédé de traitement de fibres capillaires, dans lequel on applique sur les fibres une composition comprenant au moins un pigment, au moins un composé X, au moins un composé Y, au moins un des composés X et Y étant siliconé, lesdites composé X et Y étant susceptibles de réagir ensemble par (2) une réaction de condensation, lorsqu'ils sont mis en contact les uns avec les autres; utilsation pour le traitement des fibres capillaires; et utilisation pour le gainage rémanent des fibres capillaires.

---

3. revendications: 1-3(part), 5-19(part), 30, 31-48(part)

Un procédé de traitement de fibres capillaires, dans lequel on applique sur les fibres une composition comprenant au moins un pigment, au moins un composé X, au moins un composé Y, au moins un des composés X et Y étant siliconé, lesdites composé X et Y étant susceptibles de réagir ensemble par (3) une réaction de réticulation en présence d'un preoxyde, lorsqu'ils sont mis en contact les uns avec les autres; utilsation pour le traitement des fibres capillaires; et utilisation pour le gainage rémanent des fibres capillaires.

---

EPO FORM P0402

**ANNEXE AU RAPPORT DE RECHERCHE EUROPEENNE RELATIF A LA DEMANDE DE BREVET EUROPEEN NO.** EP 07 12 3588

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-03-2008

| Document brevet cité au rapport de recherche | Date de publication | Membre(s) de la famille de brevet(s) | Date de publication |
|---|---|---|---|
| EP 1101487 A | 23-05-2001 | AU 7162100 A | 17-05-2001 |
| | | BR 0005786 A | 25-09-2001 |
| | | CA 2325745 A1 | 16-05-2001 |
| | | JP 2001139416 A | 22-05-2001 |
| | | KR 20010051701 A | 25-06-2001 |
| | | TW 226245 B | 11-01-2005 |
| | | US 6461597 B1 | 08-10-2002 |
| US 2003203978 A1 | 30-10-2003 | US 2003203979 A1 | 30-10-2003 |
| JP 11349450 A | 21-12-1999 | JP 3608942 B2 | 12-01-2005 |
| EP 0865787 A1 | 23-09-1998 | DE 69806988 D1 | 12-09-2002 |
| | | DE 69806988 T2 | 09-10-2003 |
| | | FR 2760971 A1 | 25-09-1998 |
| | | JP 10265370 A | 06-10-1998 |
| GB 2407496 A | 04-05-2005 | AUCUN | |
| WO 2007071885 A | 28-06-2007 | WO 2007071706 A2 | 28-06-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0465744 A
- EP 959066 A
- EP 1057849 A
- WO 9317060 A
- WO 9612754 A
- US 3175993 A
- US 4772675 A
- US 4871827 A
- US 4888380 A
- US 4898910 A
- US 4906719 A
- US 4962174 A
- WO 0196450 A
- FR 2679771
- EP 1184426 A
- US 6225198 B
- US 5990479 A
- US 4578266 A


### Littérature non-brevet citée dans la description


- **DONALD A. TOMALIA et al.** *Angewandte Chemie, Int. Engl. Ed.,* vol. 29 (2), 138-175
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **LANDON, S.** *Pitture e Verniei,* 1997, vol. 73 (11), 18-24
- **HUANG, MOWO.** *Pitture e Verniei,* 2000, vol. 5, 61-67
- **DABBOUSSI B.O. et al.** CdSe)ZnS core-shell quantum dots : synthesis and characterisation of a size series of highly luminescent nanocristallites. *Journal of phisical chemistry B,* 1997, vol. 101, 9463-9475
- **PENG, XIAOGANG et al.** Epitaxial Growth of highly Luminescent CdSe/CdS core/shell nanocrystals with photostability and electronic accessibility. *Journal of the American Chemical Society,* vol. 119 (30), 7019-7029
- *Cosmetics and Toiletries,* Février 1990, vol. 105, 53-64